# EUROPÄISCHE PATENTSCHRIFT

(11) **EP 2 160 990 B1**
(45) Veröffentlichungstag und Bekanntmachung des Hinweises auf die Patenterteilung: **04.11.2015**
(21) Anmeldenummer: 09001676.7
(22) Anmeldetag: 06.02.2009
(51) Int. Cl.: A61B 18/14, A61B 18/00, A61B 18/02, A61B 18/12

(54) **Versorgungsgerät mit Steuerung zum Betreiben einer Kryosonde, Kryochirurgisches Gerät**
Supply device with control for operating a cryoprobe, cry-surgical device
Appareil d'alimentation doté d'une commande destinée au fonctionnement d'une cryosonde, appareil cryochirurgical

(30) Priorität: 02.07.2008 DE 102008031298; 03.09.2008 DE 102008045563; 07.10.2008 DE 102008050635
(43) Veröffentlichungstag der Anmeldung: 10.03.2010
(73) Patentinhaber: ERBE Elektromedizin GmbH, 72072 Tübingen (DE)
(72) Erfinder: Geiselhart, Franz, 72770 Reutlingen (DE)
(74) Vertreter: Bohnenberger, Johannes

(56) Entgegenhaltungen:
- WO-A-2008/077317
- US-A1- 2004 158 237
- US-B1- 6 471 693

## Beschreibung

Die Erfindung betrifft ein Versorgungsgerät zum Betreiben einer Kryosonde und ein kryochirurgisches Gerät mit entsprechendem Versorgungsgerät gemäß der angefügten Ansprüche.

Kryochirurgische Geräte zum Applizieren eines HF-Stroms sind bekannt. Sie umfassen ein kryochirurgisches Instrument mit einer Applikationselektrode, z.B. einer HF-Ablationssonde, eine Fluidquelle und eine Steuerung zum Einstellen der Kühlleistung, die an dem kryochirurgischen Instrument bereitgestellt wird. Der HF-Strom wird appliziert, um Gewebe gezielt zu devitalisieren.

Aufgrund des HF-Stroms kommt es auch in der unmittelbaren Nachbarschaft des Instruments zu einer Austrocknung des Gewebes. Der Austrocknung wegen steigt die Impedanz zwischen dem Instrument und dem Gewebe. Der Strom, der in das Gewebe eindringen kann, bzw. der Stromeintrag nimmt ab. Fern liegende Gewebebereiche werden nicht devitalisiert. Es ist bekannt, das Instrument zur Vermeidung dieses Effekts zu kühlen, so dass eine Austrocknung des Gewebes, das die Applikationselektrode kontaktiert, vermieden wird.

Für die Bereitstellung der Kühlleistung verwendet man entweder eine Kaltdampfanlage oder den Joule-Thomson Effekt. Der Joule-Thomson Effekt tritt auf, wenn ein reales Gas oder Gasgemisch durch Drosselung (Druckänderung) eine Temperaturänderung erfährt.

Drosselt man ein Gas, etwa indem man eine Drossel einsetzt oder ein anderes Hindernis einbaut, so expandiert es. Das heißt, das vom Gas eingenommene Volumen hinter dem Hindernis nimmt zu. Dabei erhöht sich der mittlere Teilchenabstand, wodurch die Temperatur des Gases sinkt.

Die Kaltdampfanlage nutzt die Eigenschaft von Stoffen, bei unterschiedlichen Drücken unterschiedliche Siede- bzw. Kondensationstemperaturen zu haben. Die verwendeten und bevorzugten Stoffe nennt man Kältemittel. Der Arbeitsbereich einer Kaltdampfanlage ist begrenzt durch die erreichbaren Siede- bzw. Kondensationstemperaturen des Kältemittels. Üblicherweise wird ein verflüssigtes Kältemittel unter hohem Druck in eine Verdunstungskammer eingespritzt, in der ein wesentlich niedrigerer Druck vorliegt. Der Siedepunkt des entsprechenden Kältemittels hängt zum einen von dem verwendeten Kältemittel ab, zum anderen von dem in der Verdunstungskammer vorherrschenden Druck. Soweit sich also in der Verdunstungskammer ein Siedepunkt ergibt, der oberhalb der vorherrschenden Temperatur in der Verdunstungskammer liegt, verdampft das Kältemittel zumindest teilweise und entzieht dem System Wärmeenergie. Das verdampfte Kältemittel wird durch einen Ablauf wieder abgeführt und entweder erneut kondensiert oder entsorgt.

Für die effektive und vorteilhafte Verwendung von kryochirurgischen Instrumenten zur Applikation eines HF-Stroms ist es notwendig, die Temperatur der Sondenoberfläche auf einen vorgegebenen Wert einstellen zu können und bei diesem konstant zu halten.

Um eine exakte Regelung der Kühlleistung und der an der Sondenoberfläche vorherrschenden Temperatur bewerkstelligen zu können, umfassen moderne kryochirurgische Geräte Temperaturregler.

Für diese Reglung ist es notwendig, die Temperatur des Instruments zu bestimmen. Derzeit werden Miniaturmantelthermoelemente als Temperatursensoren verwendet. Diese sind relativ teuer, ihre Montage aufwändig und die Haltbarkeit aufgrund der geringen mechanischen Belastbarkeit und der Korrosionsanfälligkeit relativ gering. Für sehr dünne Sonden existieren keine Thermoelemente, die eine entsprechende Dimensionierung aufweisen. Deshalb gibt es bestimmte Sonden (z.B. Endokryosonden und Intravitrialsonden) nur ohne Temperaturregelung.

Alternativ verwendet man kryochirurgische Geräte, deren Instrumente umschaltbare Drosseln im Rücklauf enthalten. Beispielsweise haben diese Drosseln drei einstellbare Stufen. Die Temperatur lässt sich bei einer halbwegs konstanten Fluidquelle zwischen den einzelnen einstellbaren Stufen regeln. Durch ein wiederholtes Einsetzen der Instrumente oder durch ein Ausmessen der Kühlleistung lassen sich Referenzwerte ermitteln. Die so bestimmten Referenzwerte sind relativ ungenau und schwanken abhängig von dem Einsatzgebiet. Außerdem müssen für sämtliche verwendete Instrumente neue Messungen durchgeführt werden. Dies ist sehr aufwändig.

Aus der DE 10 2008 045 563, ist eine Kryosonde bekannt, mit deren Hilfe das Einstellen und Regulieren einer Sondentemperatur möglich ist, ohne auf empfindliche Temperatursensoren zugreifen zu müssen.

Aus der US 2006/0004350 ist ein Temperaturregelung durch ein Regeln eines Rücklaufdrucks im Rücklauf einer Kryosonde bekannt.

Ausgehend von diesem Stand der Technik ist es Aufgabe der vorliegenden Erfindung, ein verbessertes Versorgungsgerät zum Betreiben einer Kryosonde zur Applikation eines HF-Stroms auf ein Gewebe anzugeben. Insbesondere soll die Temperatursteuerung derart verbessert werden, dass ein hoher Stromeintrag in das Gewebe erzielt wird. Des Weiteren soll ein entsprechendes kryochirurgisches Gerät angegeben werden.

Die Aufgabe wird durch ein Versorgungsgerät gemäß dem Anspruch 1 sowie ein kryochirurgisches Gerät gemäß dem Anspruch 8 gelöst.

Insbesondere wird die Aufgabe durch ein Versorgungsgerät zum Betreiben einer Kryosonde zur Applikation eines HF-Stroms auf ein Gewebe gelöst, wobei das Versorgungsgerät umfasst:
- einen Temperaturregler zur Regelung einer Innentemperatur der Kryosonde gemäß einem Stellwert,
- eine Messeinrichtung zur Erfassung des applizierten HF-Stroms, insbesondere einer Wirkleistung des HF-Stroms,
- eine Steuerung, die dazu ausgebildet ist, den Stellwert dem Temperaturregler vorzugeben,
wobei die Steuerung zur Einstellung einer vorgegebenen Sondenoberflächentemperatur mindestens ein Messsignal von der Messeinrichtung empfängt und derart ausgebildet ist,
dass sie den Stellwert in Abhängigkeit von dem mindestens einen Messsignal wählt.

Ein wesentlicher Gedanke der vorliegenden Erfindung besteht also darin, den durch die Kryosonde applizierten HF-Strom zu erfassen und die Regelung einer Innentemperatur der Kryosonde demgemäß anzupassen. Übliche Kryosonden oder Ablationssonden umfassen einen Mantel sowie einen Expansionsbereich, in dem eine Kühlung mittels Kältemittels oder des Joule-Thomson-Effekts erfolgt. Es ist möglich, beispielsweise mittels der Lehre aus der deutschen Patentanmeldung mit der Nummer 10 2008 045 563 die Innentemperatur der Kryosonde auf einen konstanten Wert zu regeln. Hierbei wird jedoch die Wärmeleistung des HF-Stroms vernachlässigt. So kann es vorkommen, dass bei dem Anliegen eines HF-Stroms die Kühlleistung derart gering ist, dass das von der Kryosonde kontaktierte Gewebe austrocknet. Das ausgetrocknete Kontaktgewebe verhindert ein effektives Eintreten des HF-Stroms. Eine geplante Devitalisierung des Zielgewebes findet nicht statt oder beschränkt sich lediglich auf einen zu geringen Bereich.

Andererseits kann es vorkommen, dass die Innentemperatur der Kryosonden bei einem hohen HF-Strom derart hoch eingestellt wird, dass es bei der Applikation eines niedrigeren HF-Stroms zu einem Vereisen des Gewebes kommt. Auch das vereiste Gewebe hat eine deutlich erhöhte Impedanz und führt zu schlechten Behandlungsergebnissen. Erfindungsgemäß ist es möglich, den HF-Strom, und insbesondere die Wirkleistung des HF-Stroms im Gewebe, insbesondere in dem unmittelbar benachbarten Gewebe zu berücksichtigen. Somit kann der Temperaturregler derart eingestellt werden, dass auch bei einem variierenden HF-Strom eine konstante Temperatur an der Außenseite der Kryosonde, insbesondere an der Sondenoberfläche, vorliegt. Es ist möglich Temperaturänderungen im Gewebe oder an der der Kryosondenobexfläche bei der Regelung der Innentemperatur zu berücksichtigen, bevor diese einen messbaren Einfluss auf die Innentemperatur der Kryosonde haben.

Das Versorgungsgerät kann eine Auswahleinrichtung umfassen, die der Steuerung mindestens einen kryosondenspezifischen Parameter bereitstellt, insbesondere mindestens einen Parameter bezüglich des thermischen Widerstands der Kryosonde und/oder mindestens einen Parameter bezüglich der Außenfläche der Kryosonde und/oder mindestens einen Parameter bezüglich der Außenkontaktfläche der Kryosonde und/oder mindestenseinen Parameter bezüglich der Geometrie der Kryosonde und/oder mindestens einen Parameter bezüglich der Fläche einer an der Kryosonde angeordneten Elektrode. Durch das Auswerten der spezifischen Parameter und das Berücksichtigen dieser durch die Steuerung ist es möglich, von der Innentemperatur auf die Temperaturverhältnisse an der Sondenoberfläche zu schließen. So kann die Sondenoberfläche mit einer konstanten Temperatur gekühlt werden. Der kryosondenspezifischen Parameter wegen kann diese Temperatur genauer abgeschätzt werden. Eine Hysterese bei der optimalen Einstellung der Innentemperatur und/oder Oberflächentemperatur wird vermieden. Es ist möglich, sondenspezifische Modelle zu entwickeln, die die über den Sondenkörper transportierte Kühlleistung abbilden. Des Weiteren kann der Wärmeabtransport durch das Gewebe und die Blutzirkulation modelliert werden. Thermische Effekte, die beim Eintritt des HF-Stroms in das Gewebe auftreten, können bei der Vorgabe von entsprechenden Übergangsflächen ebenfalls berechnet werden. Durch ein Berücksichtigen all dieser Parameter wird ein positiver Behandlungsverlauf sichergestellt.

Die Auswahleinrichtung kann eine Selektionseinrichtung zur Erfassung des angeschlossenen Kryosondentypen umfassen. Somit kann die Detektionseinrichtung den Kryosondentyp bestimmen und an die Auswahleinrichtung kommunizieren. Die Auswahleinrichtung wählt kryosondenspezifische Parameter, wie vorab beschrieben, aus und verbessert so die Temperaturregelung.

Der Stellwert, der von der Steuerung vorgegeben wird, kann einen Rücklaufdruckstellwert umfassen, und der Temperaturregler zum Regeln des Rücklaufdrucks in einem Rücklauf der Kryosonde gemäß dem Rücklaufdruckstellwert ausgebildet sein. Das heißt, die Sondeninnentemperatur wird anhand des Rücklaufdrucks bestimmt und/oder geregelt. Vorgaben an den Temperaturregler erfolgen über Druckwerte, die zu einer bestimmten Kühlleistung führen.

Die Steuerung kann derart ausgebildet sein, dass sie bei der Auswahl des Stellwerts in Abhängigkeit von dem mindestens einen Messsignal mindestens einen thermischen Widerstand des Gewebes berücksichtigt. So kann die durch den HF-Strom generierte Erhitzung des Gewebes beim Bestimmen einer geeigneten Kühlleistung berücksichtigt werden.

Die Steuerung kann den Temperaturregler bzw. die Temperatursteuerung derart steuern, dass die Sondenoberflächentemperatur ≤ 0°C, insbesondere ≤ -3°C ist. Vorzugsweise sollte die Temperatur der Sondenoberfläche bei ca. -3°C liegen, da dann das salzhaltige Gewebe gerade noch nicht einfriert, hier aber ein maximaler HF-Stromeintrag möglich wird, der nötig ist, um fernes Gewebe zu devitalisieren. Ein Austrocknen und/oder Karbonisieren des direkt an der Elektrode anliegenden Gewebes wird vermieden.

Nachfolgend wird die Erfindung anhand einiger Ausführungsbeispiele beschrieben, die mittels Abbildungen näher erläutert werden. Hierbei zeigen:
- - Fig. 1: ein kryochirurgisches Gerät, umfassend ein Versorgungsgerät und eine Ablationssonde bei der Anwendung;
- - Fig. 2: wichtige Komponenten des Versorgungsgeräts aus Fig. 1;
- - Fig. 3: einen Temperaturregler zur Regelung der Kühlleistung der Ablationssonde; und
- - Fig. 4: einen schematischen Querschnitt durch die Ablationssonde.

In der nachfolgenden Beschreibung werden für gleiche und gleich wirkende Teile dieselben Bezugsziffern verwendet.

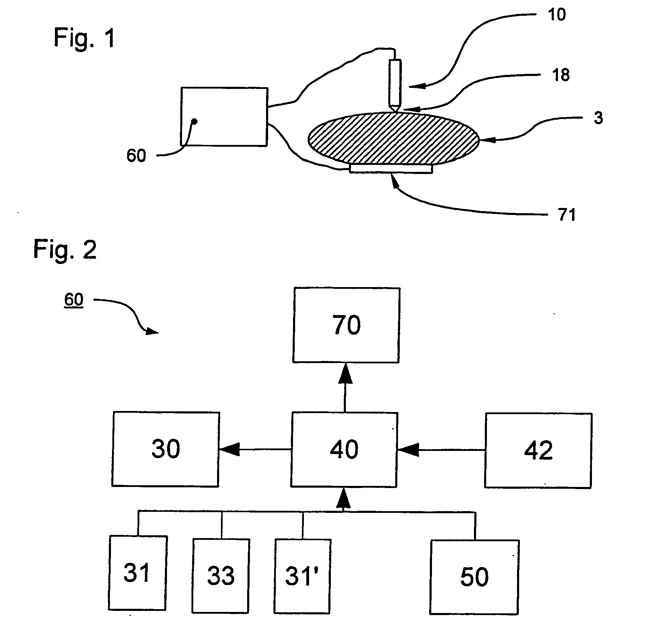

Ein erfindungsgemäßes kryochirurgisches Gerät umfasst, wie in Fig. 1 gezeigt, ein Versorgungsgerät 60, eine angeschlossene Ablationssonde 10 und eine angeschlossene Neutralelektrode 71.

Wie in Fig. 2 dargestellt, enthält das Versorgungsgerät 60 einen HF-Generator 70 zur Bereitstellung eines HF-Stroms. Über entsprechend elektrische Verbindungen wird eine HF-Spannung an eine Applikationselektrode 18 und der Neutralelektrode 71 angelegt. Die Neutralelektrode 71 ist großflächig an einem schematisch in Fig. 1 dargestellten Torso 3 angeordnet. Mittels der Ablationssonde 10 können beispielsweise Tumore devitalisiert werden. Der angelegte HF-Strom führt zu einer thermischen Erwärmung des Zielgewebes. Es ist offensichtlich, dass die Stromdichte im Gewebe mit zunehmender Distanz zur im Verhältnis zur Neutralelektrode 71 relative gering Applikationsfläche der Applikationselektrode 18 abnimmt. Eine sichere Devitalisierung des Zielgewebes ist nur in Bereichen mit einer relativ hohen Stromdichte, also nahe der Applikationselektrode 18 möglich.

Um einen maximalen HF-Stromeintrag zu erzielen und Zielgewebe mit großen Volumina zu devitalisieren, werden HF-Ströme mit einer hohen Wirkleistung appliziert. Da in unmittelbarer Nachbarschaft zu der Applikationselektrode 18 die Stromdichte üblicherweise am höchsten ist, kommt es hier zu einer Verdunstung und/oder gegebenenfalls zu einer Karbonisierung des Gewebes. Sobald dieser Effekt eintritt, steigt die Impedanz und der Stromeintrag reduziert sich. Um dies zu vermeiden, wird die Ablationssonde 10 mittels einer Kühleinrichtung gekühlt

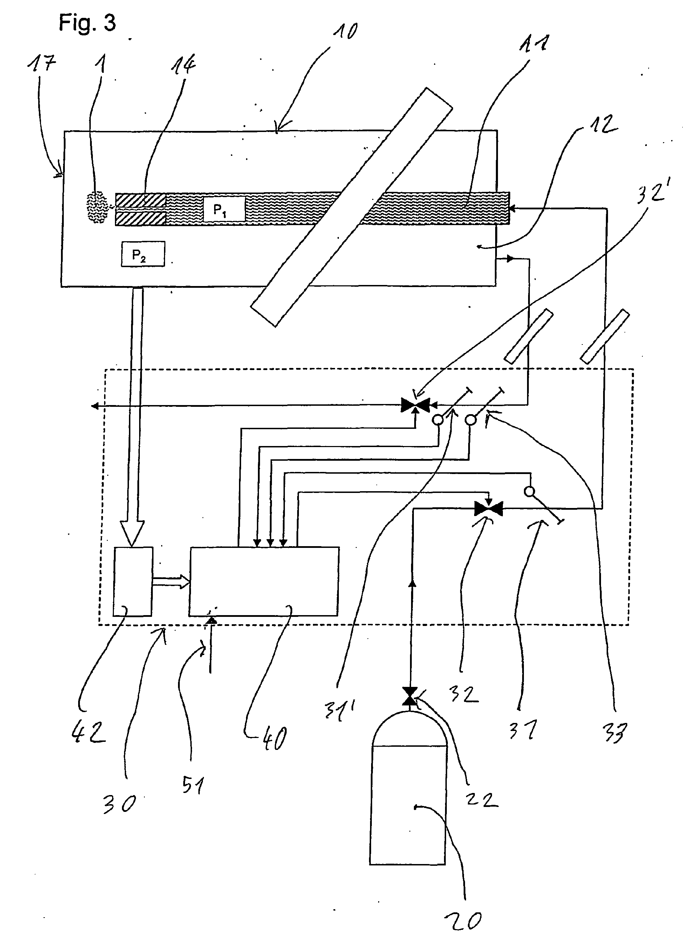

Die Kühleinrichtung umfasst eine Fluidquelle 20 (vgl. Fig. 3) zum Bereitstellen eines Kältemittels 1 mit einem vorbestimmten Druck und einen Temperaturregler 30 zur Reglung einer Innentemperatur Tᵢₙ der Ablationssonde 10. Im Inneren der Ablationssonde 10 verläuft ein Zulauf 11, der das Kältemittel 1 in einen Bereich nahe einer Sondenspitze 17 mit der Applikationselektrode 18 (in Fig. 3 nicht dargestellt) leitet. Innerhalb des Zulaufs 11 hat das Kältemittel 1 einen ersten Druck P₁, der derart hoch ist, dass der sich ergebende kältemittelspezifische Siedepunkt deutlich niedriger ist als eine Innentemperatur Tᵢₙ der Ablationssonde 10. Das Kältemittel 1 behält also seinen flüssigen Aggregatszustand. Über eine Düse 14 am distalen Ende des Zulaufs 11 wird das Kältemittel 1 in einen innerhalb der Kryosonde oder Ablationssonde 10 angeordneten Verdunstungsbereich 15 abgegeben. Der hier vorliegende zweite Druck P₂ ist deutlich niedriger als der erste Druck P₁. Somit ergibt sich kältemittelspezifisch ein niedrigerer Siedepunkt. Das Kältemittel 1 verdunstet abhängig von der in der Ablationssonde 10 vorliegenden Innentemperatur Tᵢₙ und entzieht dieser Wärmeenergie.

Alternativ kann das Kältemittel 1 in Gasform bei maximalem Betriebsdruck (Gasentnahme an der Siedelinie) derart zugeführt werden, dass das Gas bei der Abkühlung durch den Joule-Thomson Effekt an der Düse 14 sich in ein Zweiphasengemisch wandelt. Der flüssige Anteil siedet an der Innenoberfläche des Sondenkopfs bzw. der Sondenspitze 17 im Verdunstungsbereich 15.

Das Kältemittel 1 oder Gas wird dann über einen Ablauf 12 innerhalb der Kryosonde 10 in den Temperaturregler 30 zurückgeführt und von dort entsorgt oder wieder aufbereitet. Hierfür steht der Ablauf 12 in fluider Verbindung mit dem Verdunstungsbereich 15 und dem Temperaturregler 30.

Durch die Regelung des zweiten Drucks P₂ kann die Siedetemperatur und somit die Innentemperatur Tᵢₙ von der Steuerung 40 entsprechend einem vorgegebenen Wert oder Stellwert eingestellt werden. Es ist also möglich, eine direkte Korrelation zwischen dem zweiten Druck P₂ und der Kühlleistung der Ablationssonde 10 herzustellen und zu nutzen.

Um die Innentemperatur Tᵢₙ der Kryosonde 10 effektiv zu regeln, umfasst der Temperaturregler 30 einen Zulaufdruckfühler 31, einen Ablaufdruckfühler 31', ein Zulaufproportionalventil 32, ein Ablaufproportionalventil 32' und einen Durchflusssensor 33, die an die Steuerung 40 angeschlossen sind. Der Zulaufdruckfühler 31 gibt Signale aus, die es der Steuerung 40 ermöglichen, den ersten Druck P₁ im Zulauf 11 zu messen. Hierfür ist der Zulaufdruckfühler 31 an dem Zulauf 11 angeordnet. Der Ablaufdruckfühler 31' ist dementsprechend an dem Ablauf 12 angeordnet und gibt Signale aus, mittels derer die Steuerung 40 den zweiten Druck P₂ innerhalb des Verdunstungsbereichs 15 ermitteln kann. Um den Druckabfall, der beim Ausströmen des Kältemittels 1 aus dem Verdunstungsbereich 15 über den Ablauf 12 auftritt, berücksichtigen zu können, befindet sich an dem Ablauf 12 ebenfalls der Druckflusssensor 33 mittels dessen die Steuerung 40 auf den vorliegenden Strömungswiderstand schließen kann. Somit kann die Steuerung 40 den mit dem Ablaufdruckfühler 31 gemessenen Druck unter Berücksichtigung des Strömungswiderstands korrigieren und einen entsprechend bereinigten Druck P₂ ermitteln.

Diese Korrektur ist besonders wichtig, wenn die Ablationssonde 10 bei tiefen Temperaturen betrieben wird. Das zurückfließende Gas bzw. das Kältemittel 1 weist dann einen sehr niedrigen Druck P₂ und somit ein großes spezifisches Volumen auf, wodurch ein hoher Strömungswiderstand auftritt. Hinzu kommt, dass in diesem Temperaturbereich die Siedelinie von üblichen Kältemitteln 1 besonders steil verläuft und somit ein kleiner Fehler bei der Berechnung des zweiten Drucks P₂ eine starke Abweichung bei der Einstellung der Innentemperatur Tᵢₙ in der Ablationssonde 10 zur Folge hat.

Es ist ein Vorteil der vorliegenden Erfindung, dass die Sensoren und Fühler zur Bestimmung des ersten Drucks P₁ und/oder des zweiten Drucks P₂ nicht in oder nahe dem Verdunstungsbereich 15 angeordnet werden müssen.

Diese Sensoren oder Fühler können in den Ablauf 12 bzw. Zulauf 11 oder an dessen proximalen Ende angebracht sein. Somit ist eine Konstruktion von Kryosonden 10 mit deutlich geringerem Durchmesser denkbar. Alternativ können die Sensoren oder Fühler in dem Versorgungsgerät 40 angeordnet sein.

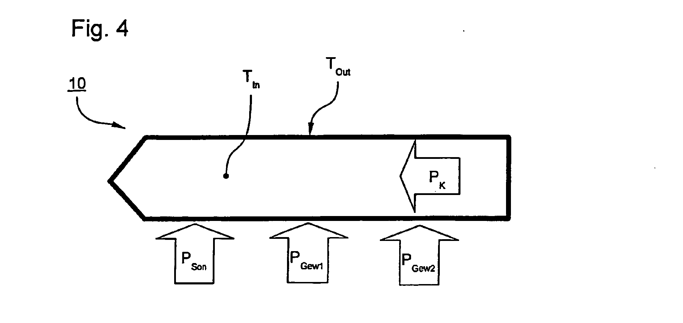

Wie in Fig. 4 schematisch verdeutlicht, kann sich die Innentemperatur Tᵢₙ der Ablationssonde 10 von einer Oberflächentemperatur Tₒᵤₜ, die an der Oberfläche des Sondenkörpers vorliegt, unterscheiden. In einem Modell kann man davon ausgehen, dass im Innenbereich der Ablationssonde 10 eine sich im Verlauf der Zeit ändernde oder konstante Kühlleistung P_{K} bereitgestellt wird. Die Kühlleistung P_{K} wirkt sich zeitlich verzögert auf die Oberflächentemperatur Tₒᵤₜ aus. Ihr steht der thermische Widerstand des Sondenmaterials entgegen.

Des Weiteren bewirkt der durch die Applikationselektrode 18 applizierte HF-Strom eine thermische Leistung P_{Son} die aufgrund der Impedanz des Sondenmaterials oder des Materials der Applikationselektrode 18' auftritt. Eine weitere thermische Leistung P_{Gew1} wird durch die Impedanz des Gewebes und den dort applizierten HF-Strom verursacht. Vorzugsweise wird die thermische Leistung P_{Gew1} berücksichtigt, die beim Eintritt des HF-Stroms in das Gewebe auftritt. Eine weitere thermische Leistung P_{Gew2} wird durch die Organe oder das Gewebe vollbracht. Auf Grund der Blutzirkulation wird das Gewebe fortlaufend beheizt.

Erfindungsgemäß soll die Ablationssonde 10 derart betrieben werden, dass an der Sondenoberfläche eine Oberflächentemperatur Tₒᵤₜ vorliegt, die ca. gleich -2°C ist. Der vorab beschriebene Temperaturregler 30 stellt Maßnahmen bereit, die Innentemperatur Tᵢₙ festzustellen und/oder einzustellen. Die erfindungsgemäße Steuerung 40 hat einen Datenspeicher, der Messreihen enthält, die die einzelnen thermischen Leistungen P_{Son}, P_{Gew1}, P_{Gew2} berücksichtigen. In diesem Datenspeicher ist mindestens ein Parameter hinterlegt, der die thermische Leistung P_{Gew2} in Abhängigkeit von der Innentemperatur Tᵢₙ angibt. Des Weiteren ist mindestens ein Parameter hinterlegt, der die thermische Verzögerung, die durch den thermischen Widerstand des Sondenmaterials vorgegeben ist, angibt. Die thermischen Leistungen Pₛₒₙ, P_{Gew1}, die von dem applizierten HF-Strom, insbesondere dessen Wirkleistung abhängen, sind in entsprechenden Messwerttabellen hinterlegt.

Die Steuerung 40 empfängt, wie in Fig. 2 gezeigt, HF-Messsignale von einer HF-Messeinrichtung 50 über einen HF-Messeingang 51 (vgl. Fig. 3). Den HF-Messsignalen entsprechend wählt die Steuerung 40 einen Datensatz aus dem Datenspeicher aus und berechnet entsprechende thermische Leistungen P_{Son} , P_{Gew1}. Anhand der thermischen Leistungen Pₛₒₙ , P_{Gew1} , P_{Gew2} und einer vorgegebenen Oberflächentemperatur Tₒᵤₜ kann eine nötige Kühlleistung P_{K} ermittelt werden.

Es ist denkbar, dass die Steuerung 40 entsprechende Werte extra- oder interpoliert. Des Weiteren ist es denkbar, Messreihen zu hinterlegen, die für eine vorgegebene Oberflächentemperatur Tₒᵤₜ und einen bestimmten HF-Strom eine Innentemperatur Tᵢₙ vorgeben. Auch hier kann inter- oder extrapoliert werden.

Des Weiteren kann, wie vorab beschrieben, die Innentemperatur Tᵢₙ über den zweiten Druck P₂ vorgegeben werden. Somit ist ein Datenspeicher denkbar, der für einen gegebenen HF-Strom, einen speziellen Sondentypen und eine gegebene Oberflächentemperatur Tₒᵤₜ eine Tabelle enthält, die den zweiten Druck P₂, als Stellwert angibt.

Eine gewünschte Oberflächentemperatur Tₒᵤₜ kann über die Dateneingabevorrichtung 42 (manuelle Eingabe) vorgegeben werden. Alternativ kann die Steuerung 40 den HF-Generator 70 und den Temperaturregler 30 derart steuern, dass die Ablationssonde 10 mit einer konstanten Oberflächentemperatur Tₒᵤₜ betrieben wird.

Die weiter oben beschriebenen Parameter bezüglich den thermischen Leistungen P_{Son}, P_{Gew1}, P_{Gew2} hängen häufig von der verwendeten Ablationssonde 10 ab. Daher umfasst ein Ausführungsbeispiel der vorliegenden Erfindung eine Detektionseinrichtung, die den Typ der an das Versorgungsgerät 60 angeschlossenen Ablationssonde 10 bestimmt. Die vorab beschriebenen Parameter können entsprechend diesem Sondentypen korrigiert werden. Alternativ ist es denkbar, eine Vielzahl von Tabellen für unterschiedliche Sondentypen zu hinterlegen.

In den beschriebenen Ausführungsbeispielen wurde zwischen dem Temperaturregler 30 und der Steuerung 40 unterschieden. Es ist möglich, dass die Steuerung 40 sämtliche Funktionen des Temperaturreglers 30 wahrnimmt, um die Innentemperatur Tᵢₙ und/oder die Oberflächentemperatur Tₒᵤₜ zu regeln oder zu steuern.

Bezüglich des vorab beschriebenen Temperaturreglers 30 sei noch angemerkt, dass in einem Ausführungsbeispiel der Strömungswiderstand allein anhand des Zulaufdruckfühlers 31 und des Ablaufdruckfühlers 31' ermittelt werden kann.

Hierfür ist es notwendig, gerätespezifische Daten über die angeschlossene Ablationssonde 10 bereitzustellen. Diese Daten umfassen beispielsweise den Querschnitt der Düse 14. Alternativ können im Labor Messungen des Strömungswiderstands abhängig von dem ersten und/oder zweiten Druck P₁, P₂ vorgenommen und tabellarisch erfasst werden. Diese gerätespezifischen Daten können ebenfalls in dem Datenspeicher hinterlegt und von der Steuerung 40 berücksichtigt werden.

Die Detektionseinrichtung kann durch das Auslesen einer Seriennummer, die in einem an der Ablationssonde 10 angebrachten RFID-Tag gespeichert ist, erfolgen.

Dem Fachmann sollten zahlreiche weitere Verfahren bekannt sein, wie eine entsprechende Identifizierung der angeschlossenen Ablationssonde 10 ermöglicht werden kann. Hierunter fallen unter anderem die Ermittlung von verschiedenen Kenngrößen in einer einer Operation vorgeschalteten Testphase der Ablationssonde 10, das Auslesen eines Bluetooth-Tags oder das Einscannen eines Barcodes.

Es sollte für den Fachmann offensichtlich sein, dass sich das Verfahren zur Regelung der Temperatur der Ablationssonde 10 auch durchführen lässt, wenn lediglich ein Ablaufdruckfühler 31' und ein Ablaufproportionalventil 32' vorhanden ist.

Die Steuerung 40 kann also nach der Ermittlung des zweiten Drucks P₂ eine Regelschleife implementieren, bei der das Ablaufproportionalventil 32', das an dem Ablauf 12 angeschlossen ist, derart eingestellt wird, dass der Druck P₂ einen vorgegebenen Wert erreicht. Dieser Wert korrespondiert abhängig von dem verwendeten Kältemittel 1 mit einer bestimmten Siede- oder Verdunstungstemperatur. Diese Temperatur kann in die Steuerung 40 durch eine Bedieneinheit oder die Dateneingabevorrichtung 42 eingegeben werden, die durch den behandelnden Arzt bedient wird.

Die Steuerung 40 kann über das optionale Zulaufproportionalventil 32 den ersten Druck P₁, der über den Zulaufdruckfühler 31 bestimmbar ist, einstellen. Somit lässt sich beispielsweise über das Zulaufproportionalventil 32 die Menge des in die Ablationssonde 10 eingebrachten Kältemittels 1 regulieren. Des Weiteren kann über das Einstellen der Druckdifferenz zwischen dem ersten Druck P₁ und dem zweiten Druck P₂ die Kühlleistung P_{K} vorgegeben werden, die mittels des Joule-Thomson Effekts erzielt wird.

Die Fluidquelle 20 verfügt ebenfalls über ein optionales Fluidquellenventil 22, das ein manuelles Einstellen des Eingangsdrucks, mit dem das Kältemittel 1 in den Temperaturregler 30 eingebracht wird, ermöglicht.

### Bezugszeichen

- 1: Kältemittel
- 3: Torso
- 10: Ablationssonde
- 11: Zulauf
- 12: Ablauf
- 14: Düse
- 15: Verdunstungsbereich
- 17: Sondenspitze
- 18: Applikationselektrode
- 20: Fluidquelle
- 22: Fluidquellenventil
- 30: Temperaturregler
- 31: Zulaufdruckfühler
- 31': Ablaufdruckfühler
- 32: Zulaufproportionalventil
- 32': Ablaufproportionalventil
- 33: Durchflusssensor
- 40: Steuerung
- 42: Dateneingabevorrichtung
- 50: HF-Messeinrichtung
- 51: HF-Messeingang
- 60: Versorgungsgerät
- 70: HF-Generator
- 71: Neutralelektrode
- P₁: Erster Druck
- P₂: Zweiter Druck
- Tᵢₙ: Innentemperatur
- Tₒᵤₜ: Oberflächentemperatur
- P_{K}: Kühlleistung
- P_{Son}: thermische Leistung wegen Impedanz der Ablationssonde
- P_{Gew1}: thermische Leistung wegen Impedanz des Gewebes
- P_{Gew2}: thermische Leistung durch Blutzirkulation

## Patentansprüche

1. Versorgungsgerät zum Betreiben einer Kryosonde (10) zur Applikation eines HF-Stroms auf ein Gewebe, umfassend:
- einen Temperaturregler (30) zur Regelung einer Innentemperatur (Tᵢₙ) der Kryosonde (10) durch ein Regeln eines Rücklaufdrucks (P₂) in einem Rücklauf (12) der Kryosonde (10) gemäß einem Rücklaufdruckstellwert,
- eine Messeinrichtung (50) zur Erfassung des mittels der Kryosonde applizierten HF-Stroms, - eine Steuerung (40), die dazu ausgebildet ist, den Rücklaufdruckstellwert dem Temperaturregler (30) vorzugeben,
wobei die Steuerung (40) zur Einstellung einer vorgegebenen Sondenoberflächentemperatur (Tₒᵤₜ) mindestens ein HF-Messsignal von der Messeinrichtung (50) empfängt und derart ausgebildet ist,
dass sie den Rücklaufdruckstellwert in Abhängigkeit von dem mindestens einen HF-Messsignal wählt, so dass der HF-Strom berücksichtigt wird.

2. Versorgungsgerät nach Anspruch 1,
**gekennzeichnet durch**
eine Auswahleinrichtung, die der Steuerung (40) mindestens einen Kryosonden (10) spezifischen Parameter, insbesondere mindestens einen Parameter bezüglich eines thermischen Widerstands der Kryosonde (10) und/oder mindestens einen Parameter bezüglich der Außenfläche der Kryosonde (10) und/oder mindestens einen Parameter bezüglich der Außenkontaktfläche der Kryosonde (10) und/oder mindestens einen Parameter bezüglich der Geometrie der Kryosonde (10) und/oder mindestens einen Parameter bezüglich der Fläche einer an der Kryosonde (10) angeordneten Elektrode (18), bereitstellt.

3. Versorgungsgerät nach Anspruch 2,
**dadurch gekennzeichnet, dass**
die Auswahleinrichtung eine Detektionseinrichtung zur Erfassung des angeschlossenen Kryosondentyps umfasst.

4. Versorgungsgerät nach einem der vorhergehenden Ansprüche,
**dadurch gekennzeichnet, dass**
die Steuerung (40) derart ausgebildet ist, dass sie bei der Auswahl des mindestens einen Rücklaufdruckstellwerts mindestens einen thermischen Widerstand des Sondenmaterials berücksichtigt.

5. Versorgungsgerät nach einem der vorhergehenden Ansprüche,
**dadurch gekennzeichnet, dass**
die Steuerung (40) den Temperaturregler (30) derart steuert, dass die Sondenoberflächentemperatur (Tₒᵤₜ) kleiner gleich 0 ° Celsius, insbesondere kleiner gleich -3 ° Celsius, ist.

6. Versorgungsgerät nach einem der vorhergehenden Ansprüche,
**dadurch gekennzeichnet, dass**
die Kryosonde (10) eine HF-Ablationssonde ist.

7. Kryochirurgisches Gerät, umfassend:
- eine Kryosonde (10) zur Applikation eines HF-Stroms auf ein Gewebe;
- ein Versorgungsgerät (60) nach einem der vorhergehenden Ansprüche.

## Claims

1. Supply device for operating a cryoprobe (10) for application of an RF current to tissue, comprising:
- a temperature regulator (30) for regulating an inner temperature (Tᵢₙ) of the cryoprobe (10) by regulation of a return pressure (P₂) in a return line (12) of the cryoprobe (10) according to a return pressure setpoint value,
- a measuring unit (50) for detecting the RF current applied by means of the cryoprobe,
- a control unit (40), which is configured to predefine the return pressure setpoint value for the temperature regulator (30), wherein the control unit (40), for setting a predefined probe surface temperature (Tₒᵤₜ), receives at least one RF measurement signal from the measuring unit (50) and is configured in such a way that it selects the return pressure setpoint value depending on the at least one measurement signal, such that the RF current is taken into account.

2. Supply device according to Claim 1, **characterized by** a selector unit which provides the control unit (40) with at least one parameter specific to the cryoprobe (10), in particular at least one parameter in respect of a thermal resistance of the cryoprobe (10) and/or at least one parameter in respect of the outer face of the cryoprobe (10) and/or at least one parameter in respect of the outer contact face of the cryoprobe (10) and/or at least one parameter in respect of the geometry of the cryoprobe (10) and/or at least one parameter in respect of the surface of an electrode (18) arranged on the cryoprobe (10).

3. Supply device according to Claim 2, **characterized in that** the selector unit comprises a detection unit for detecting the connected type of cryoprobe.

4. Supply device according to one of the preceding claims, **characterized in that** the control unit (40) is configured in such a way that, in the selection of the at least one return pressure setpoint value, it takes account of at least a thermal resistance of the probe material.

5. Supply device according to one of the preceding claims, **characterized in that** the control unit (40) controls the temperature regulator (30) in such a way that the probe surface temperature (Tₒᵤₜ) is less than or equal to 0° Celsius, in particular less than or equal to -3° Celsius.

6. Supply device according to one of the preceding claims, **characterized in that** the cryoprobe (10) is an RF ablation probe.

7. Cryosurgical device comprising:
- a cryoprobe (10) for application of an RF current to tissue;
- a supply device (60) according to one of the preceding claims.

## Revendications

1. Appareil d'alimentation destiné au fonctionnement d'une cryosonde (10) pour l'application d'un courant HF sur un tissu, comprenant :
- un régulateur de température (30) pour la régulation d'une température intérieure (Tᵢₙ) de la cryosonde (10) par un réglage d'une pression de retour (P₂) dans un retour (12) de la cryosonde (10) selon une valeur de réglage de pression de retour,
- un dispositif de mesure (50) destiné à la saisie du courant HF appliqué au moyen de la cryosonde, une commande (40), qui est constituée pour affecter la valeur de réglage de pression de retour au régulateur de température (30), pour lequel la commande (40) reçoit un signal de mesure HF du dispositif de mesure (50) pour régler une température de surface de la sonde (tₒᵤₜ) affectée et est constituée de telle manière qu'elle sélectionne la valeur de réglage de pression de retour en fonction d'au moins un signal de mesure HF de telle sorte que la courant HF est pris en considération.

2. Appareil d'alimentation selon la revendication 1,
**caractérisé en ce qu'**
un dispositif de sélection fournit à la commande (40) d'au moins une cryosonde (10) les paramètres spécifiques, en particulier au moins un paramètre concernant une résistance thermique de la cryosonde (10) et/ou au moins un paramètre concernant la surface extérieure de la cryosonde (10) et/ou au moins un paramètre concernant la surface de contact extérieure de la cryosonde (10) et/ou au moins un paramètre concernant la géométrie de la cryosonde (10) et/ou au moins un paramètre concernant la surface d'une électrode (18) disposée sur la cryosonde (10).

3. Appareil d'alimentation selon la revendication 2,
**caractérisé en ce que**
le dispositif de sélection comprend un dispositif de détection destiné à saisir le type de cryosonde connecté

4. Appareil d'alimentation selon l'une quelconque des revendications précédentes,
**caractérisé en ce que**
la commande (40) est constituée de telle manière qu'elle prend en considération lors de la sélection d'au moins une valeur de réglage de pression de retour, au moins une résistance thermique du matériau de sonde.

5. Appareil d'alimentation selon l'une quelconque des revendications précédentes,
**caractérisé en ce que**
la commande (40) commande le régulateur de température (30) de telle manière que la température de surface de sonde (Tₒᵤₜ) est inférieure à 0° Celsius, en particulier inférieur à -3° Celsius.

6. Appareil d'alimentation selon l'une quelconque des revendications précédentes,
**caractérisé en ce que**
la cryosonde (10) est une sonde d'ablation HF.

7. Appareil cryochirurgical comprenant :
- une cryosonde (10) destinée à l'application d'un courant HF sur un tissu ;
- un appareil d'alimentation (60) selon l'une quelconque des revendications précédentes.
